# FASCICULE DE BREVET EUROPEEN

(11) **EP 3 408 373 B1**
(45) Date de publication et mention de la délivrance du brevet: **01.01.2020**
(21) Numéro de dépôt: 17703353.7
(22) Date de dépôt: 27.01.2017
(51) Int. Cl.: C12N 1/20, A61K 35/64, C12R 1/46

(54) **SOUCHE BACTÉRIENNE COMME AGENTS DE PRÉVENTION ET/OU DE TRAITEMENT DE PATHOLOGIES RESPIRATOIRES**
BAKTERIENSTAMM ALS MITTEL ZUR VORBEUGUNG UND/ODER BEHANDLUNG VON ATEMWEGSERKRANKUNGEN
BACTERIAL STRAIN AS AGENTS FOR PREVENTING AND/OR TREATING RESPIRATORY DISORDERS

(30) Priorité: 27.01.2016 FR 1650656
(43) Date de publication de la demande: 05.12.2018
(73) Titulaire: Institut National De La Recherche Agronomique, 75007 Paris (FR)
(72) Inventeur: THOMAS, Muriel, 91430 Igny (FR); REMOT-BRIZION, Aude, 37360 Semblancay (FR); LANGELLA, Philippe, 78140 Vélizy (FR)
(74) Mandataire: Regimbeau
(86) Numéro de dépôt international: PCT/EP2017/051839
(87) Numéro de publication internationale: WO 2017/129787

(56) Documents cités:
- Bei Zhang ET AL: "Oral administration of Enterococcus faecalis FK-23 suppresses Th17 cell development and attenuates allergic airway responses in mice", International Journal of Molecular Medicine, 1 août 2012 (2012-08-01), pages 248-254, XP055307062, DOI: 10.3892/ijmm.2012.1010 Extrait de l'Internet: URL:https://www.spandidos-publications.com /ijmm/30/2/248/download [extrait le 2016-10-03]
- K. E. FUJIMURA ET AL: "House dust exposure mediates gut microbiome Lactobacillus enrichment and airway immune defense against allergens and virus infection", PROCEEDINGS OF THE NATIONAL ACADEMY OF SCIENCES, vol. 111, no. 2, 16 décembre 2013 (2013-12-16), pages 805-810, XP055307074, US ISSN: 0027-8424, DOI: 10.1073/pnas.1310750111
- ROBERT P. DICKSON ET AL: "The Lung Microbiome: New Principles for Respiratory Bacteriology in Health and Disease", PLOS PATHOGENS, vol. 11, no. 7, 9 juillet 2015 (2015-07-09), page e1004923, XP055307572, DOI: 10.1371/journal.ppat.1004923
- LUIS FONTANA ET AL: "Sources, isolation, characterisation and evaluation of probiotics", BRITISH JOURNAL OF NUTRITION, vol. 109, no. S2, 1 janvier 2013 (2013-01-01), pages S35-S50, XP055307574, UK ISSN: 0007-1145, DOI: 10.1017/S0007114512004011

## Description

### INTRODUCTION

On a longtemps cru que les poumons sains étaient stériles et que seules les voies aériennes supérieures étaient colonisées par des bactéries. Cependant depuis 2010, un microbiote pulmonaire a été décrit, tout d'abord chez des patients atteints d'asthme et de Broncho-Pneumopathie Obstructive Chronique (BPOC) puis chez des individus sains.

Les données disponibles dans la littérature proviennent majoritairement d'étude de métagénome [1,2, 3]. En effet, la charge bactérienne dans les poumons en bonne santé est au moins d'un ordre de grandeur plus faible que celle de l'intestin. La majorité de ces souches ne peut pas être détectée par les technologies habituelles basées sur la mise en culture des échantillons sur des milieux solides. Cependant, les progrès des technologies de séquençage ont rendu possible l'identification de souches bactériennes par le séquençage des gènes codant des ARNr 16S. il a ainsi pu être montré que le microbiote du poumon se compose d'une relativement grande diversité d'espèces bactériennes. D'un point de vue taxonomique, Le embranchements plus répandus qui ont été identifiés dans les voies respiratoires sont les proétobactéries (Proteobacteria), les Firmicutes et les Bacteroidetes. Les genre les plus fréquents sont les Pseudomonas, Streptococcus, Prevotella, Fusobacteria, Veillonella, Haemophilus ainsi que Neisseria (Hilty et al, PLoS One. 5(1):e8578, 2010; Erb-Downward et al., PLoS One. 6(2):e16384, 2011).

La colonisation par le microbiote a un impact très important sur l'immunité et la santé. La recherche dans ce domaine a été facilitée par l'utilisation de souris axéniques (sans germes). Aucun microorganisme résident du poumon n'a pour l'instant été associé à des effets bénéfiques sur l'immunité pulmonaire. Contrairement à l'intestin où la bibliographie est abondante, le microbiote bactérien pulmonaire est encore peu décrit, et son influence sur l'épithélium respiratoire et la mise en place de l'immunité chez le nouveau-né est un sujet novateur.

Les pathologies respiratoires affectent l'appareil respiratoire et provoquent des troubles de son fonctionnement. Selon les statistiques publiées en 2012 par l'Organisation Mondiale de la Santé (OMS), elles sont encore la première cause de mortalité des enfants de moins de cinq ans dans le monde en 2010. La plupart des agents pathogènes qui causent des infections respiratoires se transmettent par l'air et / ou par contact direct. Il existe un besoin dans la technique pour utiliser de façon efficace et facile des compositions pour traiter et prévenir les infections des voies respiratoires et de leurs symptômes.

Bei Zhang et al. (International Journal of Molecular Medicine, 1 août 2012, page 248-254) décrit l'utilisation d'une souche d'Enterococcus faecalis pour le traitement d'allergie pulmonaire.

### DESCRIPTION

La présente invention a pour objet de nouveaux traitements des maladies respiratoires, notamment l'asthme. La présente invention a notamment pour objet leur prévention.

Plus particulièrement, les présents inventeurs ont montré qu'une souche particulière *d'Enterococcus* sp. possède des propriétés tout à fait avantageuses dans le traitement et/ou la prévention de maladies respiratoires telles que l'asthme.

En effet, cette souche particulière limite, voire prévient, la perte de poids dans un modèle animal d'asthme allergique chronique. Dans ce modèle, les poumons des animaux qui ont reçu cette souche particulière ne présentent pas ou peu de signes d'inflammation, ce qui montre l'effet protecteur de la dite bactérie. L'épithélium pulmonaire des animaux traités a la même épaisseur que celui d'animaux sains, tandis que leurs cellules épithéliales ont un aspect très similaire à celui des cellules saines. En outre, la souche de l'invention entraine une expression augmentée de certaines cytokines Th1 mais pas celle des cytokines Th2 dont l'expression pourra même être diminuée dans certains cas.

Dans un premier aspect, l'invention a pour objet une souche *d'Enterococcus sp.* possédant des propriétés de prévention et/ou de traitement des maladies respiratoires. Plus spécifiquement, l'invention a pour objet la souche d' *Enterococcus sp.* déposée sous le numéro I-4969 le 14 avril 2015 à la Collection Nationale des Cultures de Microorganismes (CNCM), 25 rue du Docteur Roux, 75724 Paris Cedex 15, France.

La souche I-4969 est produite par culture, par exemple, dans un milieu de croissance connu de l'homme du métier (par exemple, un milieu BHI : « Brain-Heart Infusion ») pendant 2 à 3 jours, à une température de 30-37°C, avec ou sans ajustement du pH. Le bouillon de fermentation contenant les cellules bactériennes est recueilli. Le bouillon peut être utilisé tel quel, concentré ou lyophilisé. Avantageusement, les bactéries seront recueillies, par exemple par centrifugation puis remises en suspension dans un tampon approprié, par exemple du PBS (« phoshate buffered saline »). La concentration bactérienne peut être établie en utilisant un cytomètre de flux ou un autre procédé équivalent.

La souche de l'invention est particulièrement avantageuse en ce qu'elle entraine une augmentation de l'expression de cytokines Th1 mais pas celle des cytokines Th2 dont l'expression pourra même être diminuée dans certains cas. Il est en effet connu de l'homme du métier que les cytokines Th2 sont exprimées dans de nombreuses maladies respiratoires, notamment l'asthme allergique (Atamas et al., F1000 Biol Rep. 2013; 5: 3). Or il est aussi connu que les cytokines Th1 ont un effet inhibiteur sur la voie Th2. Sans vouloir être lié par la théorie, on peut penser que l'expression des cytokines Th1 induite par la bactérie de l'invention permet d'atténuer les effets des maladies respiratoires, notamment de l'asthme, par moindre activation de la réponse Th2. De fait, les inventeurs ont observé que la bactérie de l'invention n'active pas, voir diminue l'activation de la réponse Th2 et protège contre le retard de croissance induit par l'asthme.

Par « voie Th1 » ou « réponse Th1 », et par « voie Th2 » ou « réponse Th2 », on entend ici le processus de la différenciation des cellules T naïves en lymphocytes Th1 ou Th2 respectivement. On appelle « lymphocytes T CD4 Th1 » une population de lymphocytes T CD4 activés qui orientent la réponse immunitaire vers la réponse cellulaire et la cytotoxicité. De même, les « lymphocytes T CD4 Th2 » au sens de l'invention correspondent à une population de lymphocytes T CD4 activés qui orientent la réponse immunitaire vers la réponse humorale avec la production d'anticorps et, à l'extrême, la synthèse d'IgE.

Le terme « cytokine », tel qu'on l'entend ici, se rapporte à une famille de petites protéines sécrétées régulatrices ayant un rôle crucial dans les réponses immunitaires. Les cytokines sont impliquées dans la communication entre cellules et régulent de nombreuses fonctions cellulaires, comme par exemple la survie et la croissance des cellules, ainsi que l'induction de l'expression de nombreux gènes. Les cytokines peuvent être produites par de nombreux types cellulaires.

Par « cytokines Th1 », on entend ici les cytokines qui sont produites par les lymphocytes T CD4 Th1 (IL-2, IFNγ et TFNβ) ou qui sont produites par d'autres types cellulaires dans le même contexte signant une activation de la voie Th1 (IL-12p70). Par « cytokines Th2 », on entend ici les cytokines que produisent les lymphocytes T CD4 Th2 (IL-4, IL-5, IL-10 et IL-13) ou qui sont produites par d'autres types cellulaires dans le même contexte signant une activation de la voie Th2 (TSLP).

Selon la présente invention, les termes « surexpression » ou « expression augmentée » se réfèrent à une expression accrue d'un gène d'intérêt, notamment le gène d'une cytokine Th1, par rapport à celle dudit gène dans un contrôle de référence, tel que par exemple un contrôle n'ayant pas été traité avec la bactérie de l'invention. Le terme « expression », tel qu'utilisé ici, se réfère à la transcription et l'accumulation stable de sens (ARNm). L'expression comprend également la traduction de l'ARNm en un polypeptide. Le terme « augmenté », tel qu'il est utilisé ici dans certains modes de réalisation, signifie une plus grande quantité, par exemple, une quantité légèrement supérieure à la quantité d'origine ou par exemple une quantité en grand excès par rapport à la quantité d'origine, et notamment toutes les quantités dans l'intervalle. En variante, « augmentation » peut faire référence à une quantité ou une activité qui est au moins 1%, 2%, 3%, 4%, 5%, 6%, 7%, 8%, 9%, 10%, 11%, 12%, 13%, 14%, 15%, 16%, 17%, 18%, 19% ou 20% de plus que la quantité ou de l'activité pour laquelle la quantité ou de l'activité accrue est comparé. Les termes « augmenté », « plus grand que », et « accru » sont utilisés ici de manière interchangeable.

Par « diminution de la réponse Th2 » ou « moindre activation de la réponse Th2 », il est entendu ici une diminution de l'expression d'un gène d'intérêt, notamment le gène d'une cytokine Th2, par rapport à celle dudit gène dans un contrôle de référence, tel que par exemple un contrôle n'ayant pas été traité avec la bactérie de l'invention. Le terme « diminution », tel qu'il est utilisé ici dans certains modes de réalisation, signifie une plus petite quantité, par exemple, une quantité légèrement inférieure à la quantité d'origine, ou par exemple une quantité beaucoup plus petite que la quantité d'origine, et notamment toutes les quantités dans l'intervalle. En variante, « diminution » peut faire référence à une quantité ou une activité qui est au moins 1%, 2%, 3%, 4%, 5%, 6%, 7%, 8%, 9%, 10%, 11%, 12%, 13%, 14%, 15%, 16%, 17%, 18%, 19% ou 20% de moins que la quantité ou de l'activité pour laquelle la quantité ou de l'activité diminuée est comparé. Les termes « diminué », « plus petit que », « moindre » et « décru » sont utilisés ici de manière interchangeable.

Ledit « contrôle » tel qu'utilisé ici peut être un patient, un modèle animal ou encore un modèle cellulaire in vitro. De préférence, le « contrôle » est un patient. Par « patient », on entend ici un sujet humain souffrant d'une maladie respiratoire, notamment d'asthme.

Dans un autre aspect, la présente invention a aussi pour objet une composition pharmaceutique comprenant la souche I-4969 et au moins un excipient pharmaceutiquement acceptable.

La bactérie inactivée induit les mêmes effets que la souche vivante et possède donc elle aussi des propriétés de prévention et/ou de traitement des maladies respiratoires.

Selon un mode de réalisation particulier de l'invention, la souche I-4969 présente dans la composition pharmaceutique est une souche inactivée. Par « souche inactivée », on entend ici une souche bactérienne qui est incapable de croître et/ou de se diviser. Préférentiellement, une souche inactivée n'a plus d'activité métabolique. Toutefois, les bactéries inactivées selon l'invention sont toujours capables de modérer la voie d'activation Th2, c'est-à-dire que l'administration de la bactérie inactivée entraine une diminution de l'activation de la voie Th2.

Les techniques d'inactivation des bactéries sont bien connues de l'homme du métier. On citera par exemple l'inactivation par la chaleur, l'irradiation par les UV ou les rayons gamma, le traitement par les acides, le traitement par le peroxyde d'hydrogène, etc. Les bactéries selon l'invention seront préférentiellement inactivées par traitement à la chaleur.

Il est particulièrement avantageux d'utiliser des extraits de la souche I-4969 dans les compositions pharmaceutiques de l'invention. Un « extrait », tel qu'on l'entend ici, consiste en un lysat de la souche I-4969.

La lyse peut être réalisée par tous les moyens connus de l'homme du métier : lyse alcaline, lyse par sonication, lyse par haute pression (presse de French), etc.

Les compositions de l'invention sont utiles pour le traitement des maladies respiratoires. Les termes « traiter », « traité », « traitement », ainsi que les termes analogues, tels qu'ils sont utilisés ici, se réfèrent à la réduction ou l'amélioration des symptômes d'un trouble (par exemple, une maladie respiratoire, notamment l'asthme) et/ou les symptômes associés avec celui-ci chez un sujet. On notera que, bien que ce ne soit pas exclu, le traitement d'un trouble ou un état ne nécessite pas que la pathologie, la condition ou les symptômes qui lui sont associés soient complètement éliminés.

Les termes « prévenir », « prévention», ainsi que les termes analogues, tels qu'ils sont utilisés ici, se réfèrent à la suppression du risque d'apparition d'un trouble (par exemple, une maladie respiratoire, notamment l'asthme) et/ou les symptômes associés avec celui-ci chez un sujet.

On entend ici par «sujet» n'importe quel mammifère pouvant bénéficier du traitement décrit ici, y compris l'homme, le chien, le chat, les bovins, les caprins, les porcs, les ovins et les primates non-humains. Plus spécifiquement, on appelle ici « patient » un sujet humain. Ledit patient peut appartenir à n'importe quelle classe d'âge, c'est-à-dire le patient peut être un enfant, un adolescent ou un adulte. Il est connu que les enfants sont plus sensibles aux maladies respiratoires, notamment l'asthme. Dans un mode préféré de réalisation, le patient selon l'invention est un enfant.

Par « maladie respiratoire » au sens de l'invention, on entend ici les maladies de l'appareil respiratoire ou provoquant des troubles de la respiration.

Plus particulièrement, les compositions de l'invention sont utiles pour le traitement et/ou la prévention de troubles respiratoires inflammatoires, par exemple l'asthme (légère, modérée ou sévère), par exemple, bronchique, allergique, intrinsèque, extrinsèque, induite par l'exercice, d'origine médicamenteuse (y compris l'aspirine et aux AINS) et l'asthme induit par la poussière, l'asthme résistant aux stéroïdes, la bronchite, y compris la bronchite infectieuse et à éosinophiles, d'une maladie pulmonaire obstructive chronique (COPD), comme la BPCO (broncho-pneumopathie chronique obstructive), la fibrose cystique, la fibrose pulmonaire, y compris cryptogénique alvéolite fibrosante, la fibrose pulmonaire idiopathique, prieumonias interstitielles idiopathiques, la fibrose compliquant une thérapie anti-néoplasique et chronique l'infection, y compris la tuberculose et l'aspergillose et d'autres infections fongiques; complications de la transplantation pulmonaire; vascularite et troubles thrombotiques du système vasculaire pulmonaire et hypertension artérielle pulmonaire (y compris l'hypertension artérielle pulmonaire); activité antitussive comprenant le traitement de la toux chronique associée à des affections inflammatoires et sécrétoires des voies respiratoires et la toux iatrogène; rhinite aiguë et chronique, y compris la rhinite médicamenteuse, et la rhinite vasomotrice; perannuelle et saisonnière rhinite allergique y compris la rhinite nerveuse (rhume des foins); polypose nasale; infection aiguë virale, y compris le rhume, et l'infection due au virus respiratoire syncytial, la grippe, coronavirus (SRAS notamment) et l'adénovirus, un oedème pulmonaire, embolie pulmonaire, pneumonie, sarcoïdose pulmonaire, la silicose, poumon de fermier et les maladies apparentées; pneumopathie d'hypersensibilité, insuffisance respiratoire, syndrome de détresse respiratoire aiguë, l'emphysème, la bronchite chronique, la tuberculose et le cancer du poumon.

En particulier, les utilisations et les compositions de la présente invention englobent la prévention et le traitement de l'asthme, de la BPCO et de la rhinite. Encore plus particulièrement, les utilisations et les compositions de la présente invention portent sur la prévention et le traitement de l'asthme. Les inventeurs ont démontré que la souche I-4969, même quand elle est inactivée par la chaleur, est particulièrement efficace pour prévenir l'asthme lorsqu'elle est administrée avant l'apparition de l'ensemble de symptômes de la maladie. Ainsi, l'utilisation de la souche de l'invention et la composition la comprenant pourront notamment être utilisées efficacement pour prévenir l'apparition de l'asthme chez des sujets connus pour leur prédisposition pour cette pathologie (par exemple, des sujets ayant une prédisposition familiale au développement de l'asthme).

On entend ici par « asthme » une maladie pulmonaire inflammatoire chronique qui se caractérise par l'obstruction réversible des voies respiratoires. Plus particulièrement, l'asthme est un syndrome inflammatoire chronique de la muqueuse bronchique dans lequel de nombreuses cellules jouent un rôle (en particulier les mastocytes, les éosinophiles et les lymphocytes T) dans un processus en cascade. L'inflammation provoque la contraction des muscles lisses bronchiques (bronchoconstriction) associée au gonflement des parois. Cela conduit à une réduction du diamètre des bronches, d'où une limitation du flux aérien qui est, au moins en partie, réversible (soit spontanément, soit par action thérapeutique). L'inflammation cause également une augmentation associée de la réactivité des voies aériennes à une diversité de stimuli. En outre, l'inflammation entraîne une hypersécrétion de mucus. La maladie se manifeste par des crises, sous forme de sifflements, de toux et d'épisodes de gêne respiratoire sifflante (dyspnée).

Les compositions pharmaceutiques de l'invention comprennent outre la souche I-4969, un ou plusieurs excipients pharmaceutiquement acceptables.

Par « excipient pharmaceutiquement acceptable », on entend ici un excipient dont l'administration à un individu ne s'accompagne pas d'effets délétères significatifs. Les excipients pharmaceutiquement acceptables sont bien connus de l'homme du métier.

Tel qu'il est utilisé ici, l'expression « excipient pharmaceutiquement acceptable » comprend tous les solvants, tampons, solutions salines, milieux de dispersion, revêtements, agents antibactériens et antifongiques, agents isotoniques et retardant d'absorption, et analogues qui sont physiologiquement compatibles. Les excipients sont choisis selon la forme pharmaceutique et le mode d'administration souhaité, parmi les excipients habituels qui sont connus de l'homme du métier. Le type de support sera ainsi choisi en fonction de la voie d'administration prévue. Dans divers modes de réalisation, le support est approprié pour une administration intraveineuse, intrapéritonéale, sous-cutanée, intramusculaire, topique, transdermique ou orale. Les véhicules pharmaceutiquement acceptables comprennent les solutions ou dispersions aqueuses stériles et des poudres stériles pour la préparation extemporanée de solutions ou dispersions injectables stériles. L'utilisation de milieux et agents pour des substances pharmaceutiquement actives est bien connue dans la technique. Une composition pharmaceutique typique pour une perfusion intraveineuse pourrait être constituée pour contenir 250 ml de solution de Ringer stérile et 100 mg de la combinaison. Les procédés pour préparer des composés administrables par voie parentérale seront connus ou évidents pour l'homme du métier et sont décrits plus en détail dans, par exemple, « Remington's Pharmaceutical Science, 17th ed., Mack Publishing Company, Easton, Pa. (1985) », et le 18e et 19e éditions de ce manuel.

Les compositions de l'invention sont administrées au patient à une dose thérapeutiquement efficace. Le terme « dose thérapeutiquement efficace » tel qu'utilisé ici se réfère à la quantité nécessaire pour observer une activité thérapeutique ou préventive sur la maladie respiratoire, notamment l'asthme, en particulier la quantité nécessaire pour observer une amélioration des symptômes. La quantité de bactérie I-4969 à administrer ainsi que la durée du traitement sont évaluées par l'homme du métier selon l'état physiologique du sujet à traiter, la nature de ou des articulations arthrosiques à traiter, le peptide choisi ainsi que la voie d'administration utilisée. La souche bactérienne utilisée selon l'invention peut être administrée sous la forme d'une dose unique ou de doses multiples.

L'homme du métier saura ainsi choisir au mieux les voies et modes d'administration de la composition selon l'invention, ainsi que les posologies et formes galéniques optimales, selon les critères généralement pris en compte dans la fabrication d'un médicament ou l'établissement d'un traitement pharmaceutique ou vétérinaire. De préférence, ces composés seront administrés par voie systémique, en particulier par voie intraveineuse, par voie intramusculaire, intradermique, intra-péritonéale ou sous-cutanée, par voie orale, ou par voie topique (au moyen de gel, aérosols, gouttes, etc.). Les formes unitaires d'administration appropriées comprennent les formes par voie orale telles que les comprimés, les gélules molles ou dures, les poudres, les granules et les solutions ou suspensions orales, les formes d'administration sublinguale, buccale, intra-trachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Il sera particulièrement avantageux selon l'invention d'administrer la composition par voie entérale, orale, parentérale (par exemple sous-cutanée, intradermique, ou intramusculaire) ou mucosale (par exemple intranasale, sublinguale, intravaginale, transcutanée). De manière plus préférée, la composition pharmaceutique de l'invention sera administrée à plusieurs reprises, de manière étalée dans le temps. Son mode d'administration, sa posologie et sa forme galénique optimale peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

Dans les compositions pharmaceutiques de la présente invention, le principe actif ou les principes actifs sont généralement formulés en unités de dosage. Par exemple, quand la bactérie I-3699 vivante est administrée, l'unité de dosage contient entre 10² et 10⁵ ufc, avantageusement entre 10³ et 10⁵ ufc, de préférence de 10³ à 10⁴ ufc par unité de dosage, pour les administrations quotidiennes, une ou plusieurs fois par jour. Par ailleurs, quand des extraits bactériens sont administrés au patient, l'unité de dosage contient 2,5 à 500 mg, avantageusement de 10 à 250 mg, de préférence de 10 à 150 mg par unité de dosage, pour les administrations quotidiennes, une ou plusieurs fois par jour. Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

L'invention sera décrite plus précisément au moyen des exemples ci-dessous.

### LEGENDES DES FIGURES

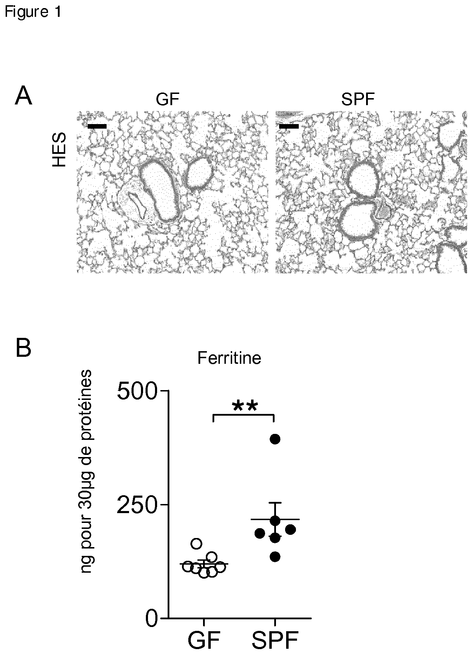

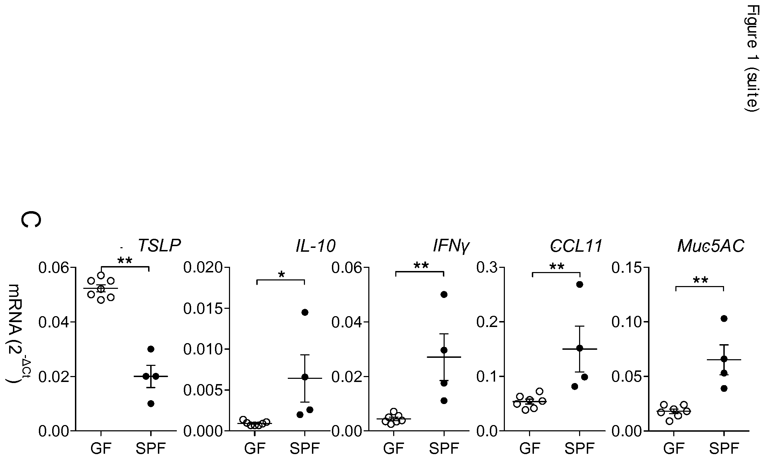

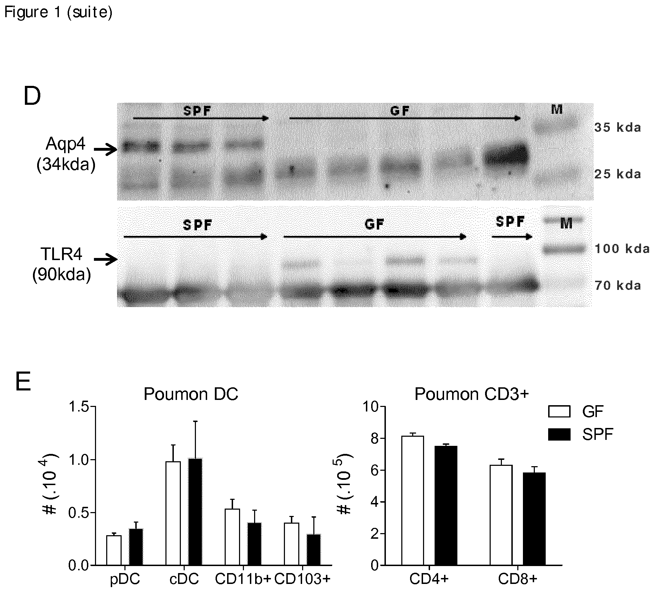

Figure 1. Les poumons GF (*germ-free*) sont totalement fonctionnels malgré une homéostasie différente.
   (A) Les poumons ont été fixés, enrobés de paraffine et coupés par section de 5 µm. Les sections de poumon ont été colorées avec l'hématoxyline éosine safran (HES) et photographiées à l'aide d'un scanner de lame et du logiciel CaseViewer. Une section représentative est montrée par groupe.
   (B) La ferritine a été dosée dans 30µg de protéines totales de poumon. Les données sont montrées individuellement et en tant que moyenne ± écart type.
   (C) Le niveau d'ARNm est exprimé en tant que 2-ΔCt, calculé à partir du Ct (cycle seuil) de l'ARNm rapporté au Ct de l'ARNm de HPRT (ΔCt). Les données sont montrées individuellement et en tant que moyenne ± écart type.
   (D) Western Blot anti Aquaporine 4 et TLR4 à partir de protéines de poumons.
   (E) Les cellules de poumon ont été marquées avec un anticorps monoclonal (mAb) ou un contrôle isotypique et analysées par cytométrie de flux. Les cellules dendritiques plasmacytoïdes (pDC) ont été identifiées comme des cellules CD11^{c+} MHCII⁺ mPDCA1⁺ CD11b⁻. Les cellules dendritiques ont été analysées par sélection des cellules CD11c+ parmi les CLH II^{high} et analyse de l'expression de CD11b et CD103, ce qui permet de définir les deux sous-groupes principaux des cellules dendritiques: CD11b⁺ CD103⁻ (CD11b) et CD11b⁻ CD103⁺ (CD103). Les cellules T ont été identifiées comme des cellules CD3⁺ CD4⁺ or CD3⁺ CD8⁺. Les données correspondent à la moyenne ± écart type de n ≥ 6 souris.

   Toutes les données ont été obtenues dans deux expériences indépendantes.

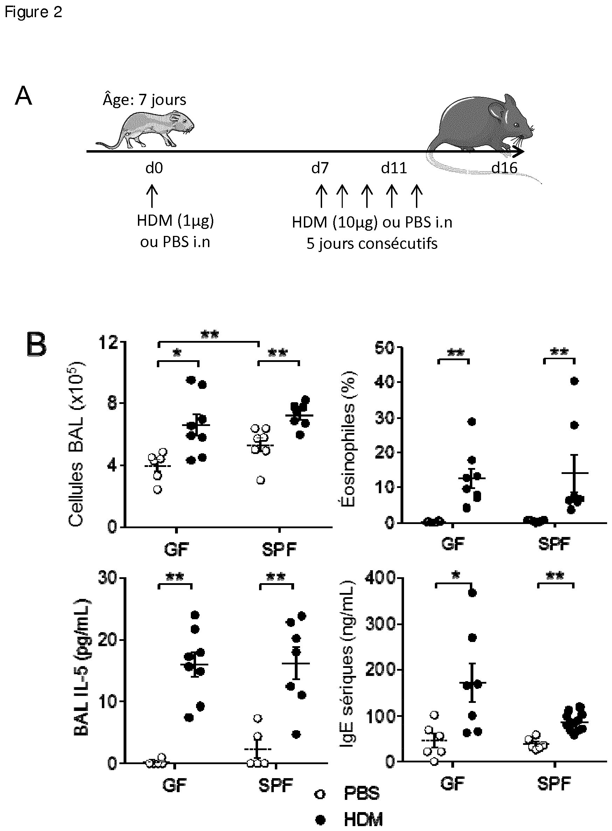

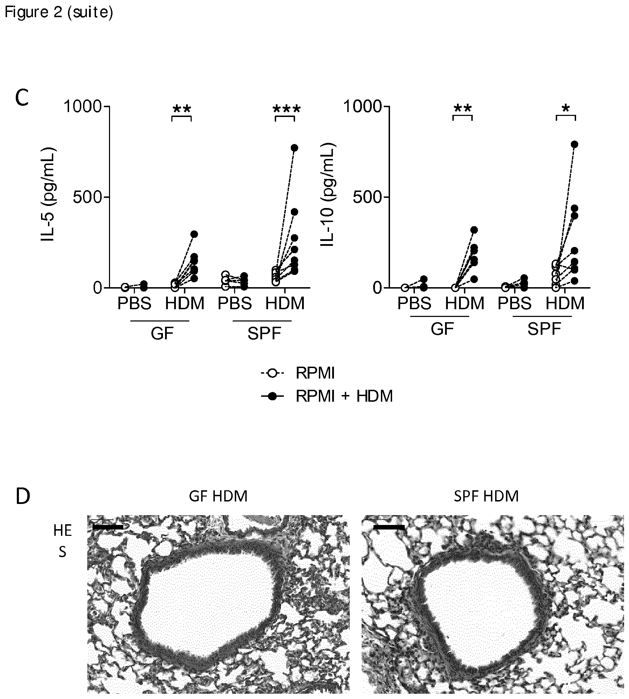

Figure 2. L'asthme induit par HDM n'est pas exacerbé dans les souris GF.
   (A) Protocole pour induire l'asthme avec HDM.
   (B) Les cellules présentes dans le lavage broncho-alvéolaire (BAL) ont été dénombrées, cytocentrifugées et colorées avec le May-Gründwald-Giemsa. Les éosinophiles ont été dénombrés et sont exprimés en % des cellules BAL totales. Les niveaux de la cytokine IL-5 dans le BAL et d'IgE sériques ont été mesurés par ELISA. Les données correspondent à la moyenne ± écart type.
   (C) Les cellules de chaque ganglion lymphatique respiratoire (RLN) ont été isolées et cultivées 72h en RPMI avec ou sans HDM. Les niveaux dans le surnageant des cytokines IL-5 et IL-10 ont été mesurés par ELISA. Les valeurs RPMI ± HDM pour une même souris sont reliées par un trait pointillé.
   (D) L'histologie des poumons a été réalisée comme décrit pour la Figure 1. Une section représentative est montrée par groupe.

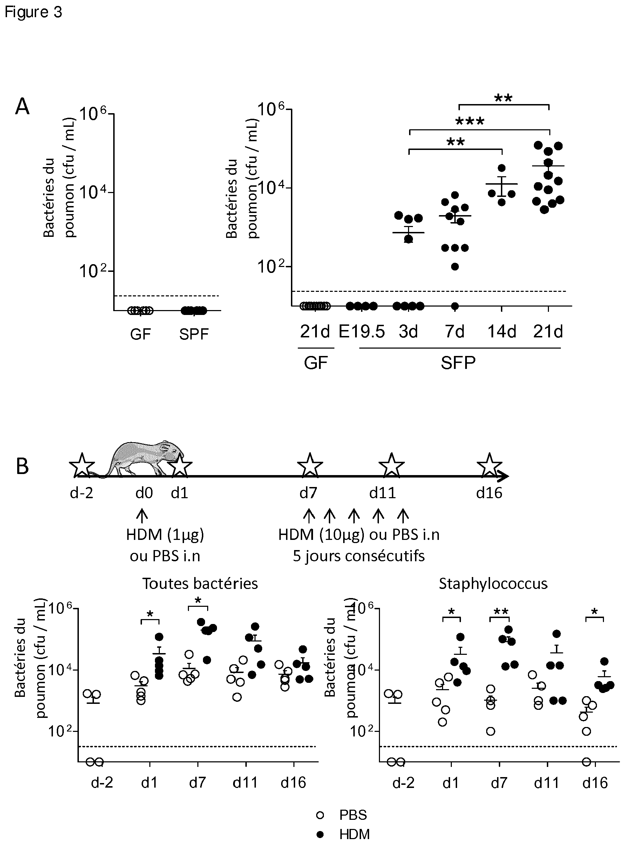

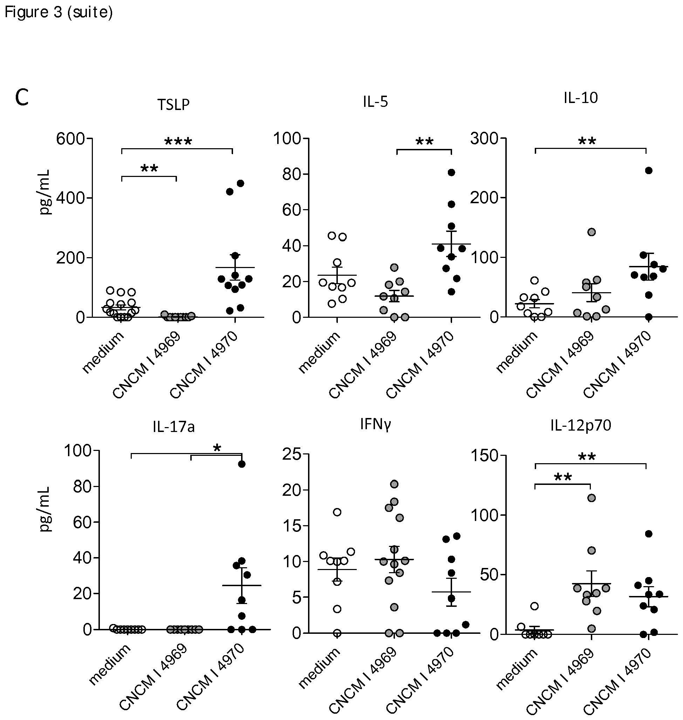

Figure 3. Le microbiote du poumon est modifié pendant l'asthme induit par HDM.
   (A) Des homogénats BAL ou de poumon ont été incubés 24h sur des boites yhBHI et les unités formant colonie (ufc) ont été dénombrées par mL de BAL ou par g de poumon.
   (B) Schéma expérimental du protocole et résultats de dénombrement bactérien des poumons à différents jours du protocole. Des homogénats de poumon ont été incubés 24h sur des boites yhBHI et les unités formant colonie (ufc) totales ou de Staphylococcus ont été dénombrées par g de poumon.
   (C) Les souches ont été co-incubées en présence d'explants de poumon. Les cytokines IL-5, IL-10, IL-12p70, IL-17a, IFNγ et TSLP ont été quantifiées par ELISA en suivant les instructions du fabricant. « Médium » : contrôle des milieux de culture sans bactéries.

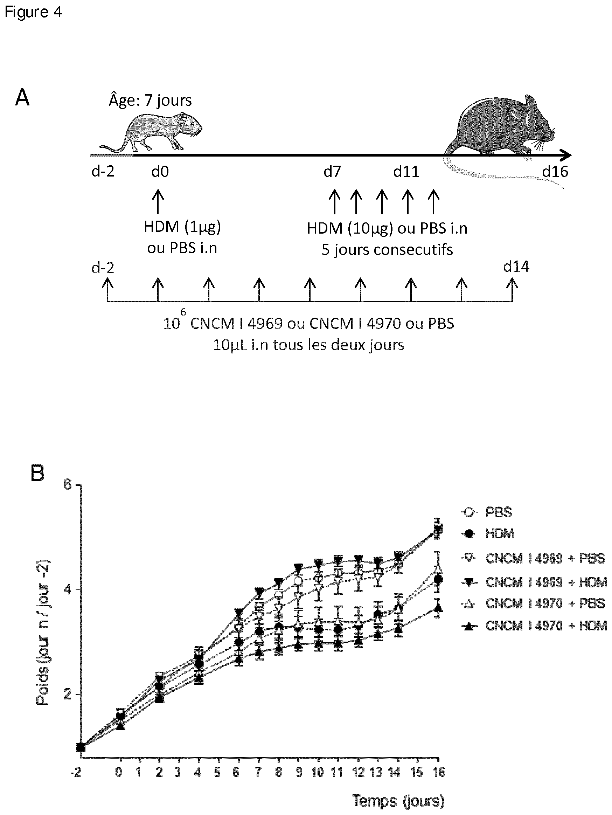

Figure 4. L'intervention bactérienne peut moduler les caractéristiques de l'asthme.
   (A) Schéma expérimental du protocole
   (B) Les souris sont pesées quotidiennement. Les courbes de croissance correspondent à la moyenne ± écart type du poids d'une souris (normalisé au poids initial au jour-2). La méthode de Tukey pour les comparaisons multiples dans l'ANOVA a été utilisée pour comparer les courbes de croissance.
Figure 5. La signature Th2 est diminuée par CNCM I 4969.
   (A) Les cellules présentes dans le lavage broncho-alvéolaire (BAL) ont été dénombrées, cytocentrifugées et colorées avec le May-Gründwald-Giemsa. Les éosinophiles ont été dénombrés et sont exprimés en % des cellules BAL totales. 4969
   (B) Les niveaux de la cytokine IL-5 dans le BAL et d'IgE sériques ont été mesurés par ELISA. Les données correspondent à la moyenne ± écart type.
   (C) Les cellules de chaque ganglion lymphatique respiratoire (RLN) ont été isolées et cultivées 72h en RPMI avec ou sans HDM. Les niveaux dans le surnageant des cytokines IL-5 et IL-10 ont été mesurés par ELISA. Les valeurs RPMI ± HDM pour une même souris sont reliées par un trait pointillé.
Figure 6. CNCM I 4969 protège de l'inflammation du poumon alors que CNCM I 4970 l'accroît.
   (A) Des sections de poumons de 5 µm ont été colorées avec l'hématoxyline éosine safran (HES) ou avec le bleu Alcian/réactif de Schiff à l'acide périodique (PAS).
   (B) L'épaisseur des épithéliums de poumon ont été mesurées pour chaque section de poumon.
Figure 7. Quand elles sont administrées ensemble, CNCM I 4969 et 4970 induisent un profil d'inflammation intermédiaire.
   (A) Les souris sont pesées quotidiennement. Les courbes de croissance correspondent à la moyenne ± écart type du poids d'une souris (normalisé au poids initial au jour-2). La méthode de Tukey pour les comparaisons multiples dans l'ANOVA a été utilisée pour comparer les courbes de croissance.
   (B) Les cellules présentes dans le lavage broncho-alvéolaire (BAL) ont été dénombrées, cytocentrifugées et colorées avec le May-Gründwald-Giemsa. Les éosinophiles ont été dénombrés et sont exprimés en % des cellules BAL totales.
   (C) Les cellules de chaque ganglion lymphatique respiratoire (RLN) ont été isolées et cultivées 72h en RPMI avec ou sans HDM. Les niveaux dans le surnageant de la cytokine IL-5 ont été mesurés par ELISA. Les valeurs RPMI ± HDM pour une même souris sont reliées par un trait pointillé.
   (D) Des sections de poumons de 5 µm ont été colorées avec l'hématoxyline éosine safran (HES) ou avec le bleu Alcian/réactif de Schiff à l'acide périodique (PAS).

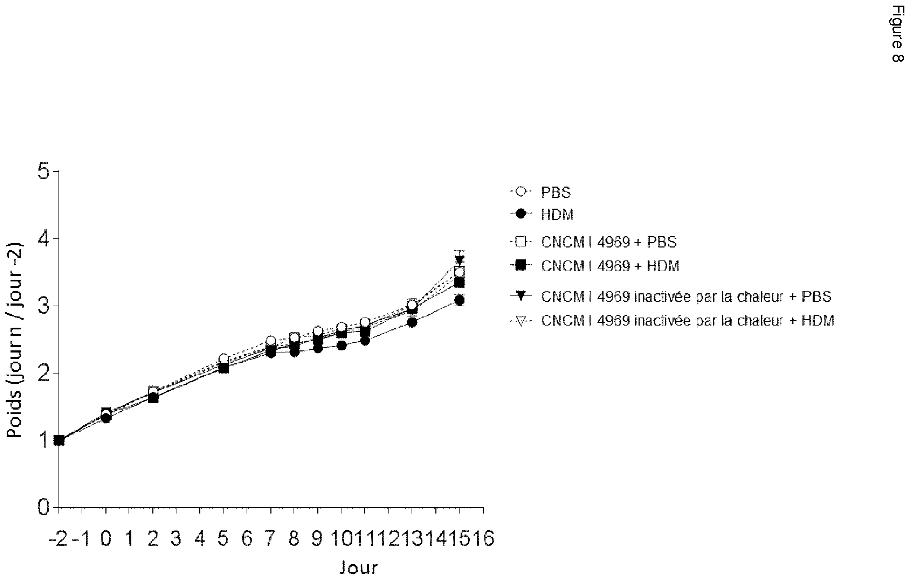

Figure 8. La souche bactérienne CNCM I 4969 inactivée par la chaleur favorise la prise de poids des souris asthmatiques et les protègent ainsi contre le retard de croissance dû à l'asthme.
   Les souris sont pesées quotidiennement. Les courbes de croissance correspondent à la moyenne ± écart type du poids d'une souris (normalisé au poids initial au jour-2). La méthode de Tukey pour les comparaisons multiples dans l'ANOVA a été utilisée pour comparer les courbes de croissance.

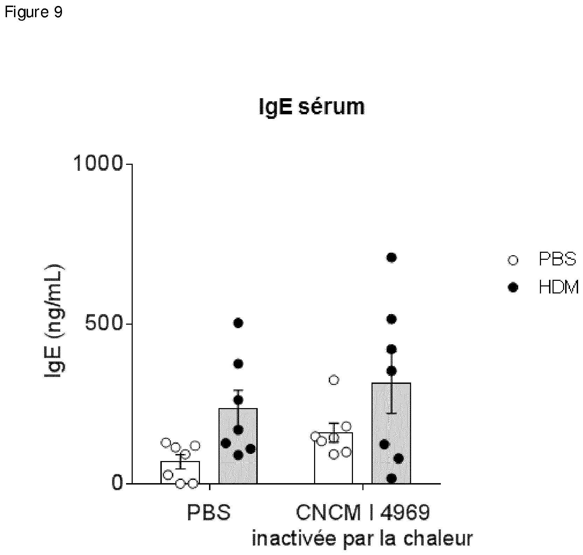

Figure 9. La souche bactérienne_CNCM I 4969 inactivée par la chaleur n'induit pas une diminution des IgE sanguins.

Le niveau de la cytokine IgE sérique a été mesuré par ELISA. Les données correspondent à la moyenne ± écart type.

### EXEMPLES

### 1. Procédures Expérimentales

### • Effet sur l'évolution de l'asthme chez des souris inoculées avec la souche bactérienne CNCM I 4969

### Souches bactériennes, milieux, conditions de croissance

Les souches bactériennes pulmonaires ont été isolées d'homogénats de poumons de souris avec un homogénéiseur (Ultraturax (IKA) ou Tissu Lyser (Qiagen)). Elles ont ensuite été cultivées sur milieu yhBHI, M17, MRS, ou Mannitol Sel Agar pendant 24 à 48h à 37°C sous conditions aérobie ou 5 jours à 37°C dans une chambre Freter sous conditions anaérobie. Les souches isolées ont été congelées à -80°C dans 16% de glycérol. L'identité de chaque souche a été confirmée par spectrophotométrie de masse et séquençage par PCR de l'ARN 16S. Les souches sélectionnées ont été déposées à la Collection Nationale des Cultures de Microorganismes (CNCM). La souche de l'invention a ainsi été déposée sous la référence CNCM I 4969. Il est à noter que cette souche a originellement été caractérisée comme étant un streptocoque. Toutefois, des études ultérieures par spectrophotométrie de masse ont permis de montrer que cette bactérie fait partie de la famille *Enterococcus* sp.qui est très proche d'un streptocoque. Par ailleurs, une souche *Staphylococcus sciuri* a été isolée dans le même crible (CNCM I 4970).

### Animaux et procédures y relatives

Les présentes expériences sur les animaux ont été approuvées par le comité d'éthique COMETHEA sous le numéro 01553.01. SPF Les souris C57BL/6 ont été obtenues de Janvier (Le Genest, St Isle, France). Elles ont été croisées et élevées en conditions FELASA SPF dans nos animaleries (IERP, INRA, Jouy-en-Josas or VIB, Gent). Les souris GF C57BL/6 ont été obtenues de CDTA (CNRS, Orleans, France) ou par reproduction en interne (INRA, Jouy-en-Josas). Elles ont été croisées et élevées en conditions stériles dans des isolateurs de type Trexler (La Calhène, Vélizy, France) dans l'animalerie Anaxem (INRA, Jouy-en-Josas). Pour l'induction de l'asthme allergique par HDM, des souriceaux de 7 jours ont reçu 1 µg de HDM (Greer) ou du PBS sans LPS (Lonza) dans un volume total de 10 µL. Une semaine après, ils recevaient 10µg de HDM (ou du PBS) pendant 5 jours consécutifs. Dans certaines expériences, des souriceaux de 5 jours ont reçu 1.10⁶ bactéries en PBS dans un volume total de 10µL, tous les deux jours.

### Prélèvements d'échantillons

Les souris ont été sacrifiées par overdose de kétamine et de xylazine. Le lavage broncho-alvéolaire (BAL) a été réalisé sur les lobes droits avec du PBS 1 mM EDTA comme décrit précédemment (Roux et al., 2011). Les surnageants BAL ont été congelés et conservés à - 20°C. Les cellules BAL ont été cytocentrifugées (Cytospin 5) sur des lames de microscope (Superfrost), puis colorées avec le May-Grünwald et le Giemsa. Le lobe droit a été utilisé pour la cytométrie de flux ou pour dénombrer les bactéries sur boites de milieu gélifié après homogénéisation avec un Tissue Lyser. Pour la cytométrie de flux, le lobe droit et les ganglions lymphatiques respiratoires (RLN) (cervicaux, mastoïdiens et médiastinaux) ont été traités avec 1 mg/mL de collagénase D et 0,5 mg/mL de DNAse I pour isoler les cellules. Le lobe gauche du poumon a été gardé à -80°C jusqu'à ce qu'il soit utilisé pour extraire l'ARN. Alternativement, les poumons ont été fixés avec le paraformaldéhyde (PFA) 4% et enrobés de paraffine, avant l'examen histologique.

### Histologie

Des sections de poumons de 5 µm ont été colorées avec l'hématoxyline éosine safran (HES) ou avec le bleu Alcian/réactif de Schiff à l'acide périodique (PAS) et photographiées à l'aide du programme du logiciel CaseViewer.

### Analyse de l'expression des gènes par q-RT-PCR

L'ARN total a été extrait des homogénats de poumon à l'aide du kit NucleoSpin® RNA (Macherey Nalgen). Les ADNc ont été obtenus par rétro-transcription en utilisant des amorces aléatoires et la reverse transcriptase (kit High-Capacity cDNA Archive Kit, Applied Biosystems by Life Technologies SAS, Saint Aubin, France) selon les instructions du fabricant. Les amorces utilisées (Sigma-Aldrich, Eurogenetec) sont listées dans le Tableau Supplémentaire 1. La réaction de q-RT-PCR a été répétée trois fois pour chaque gène en utilisant le système AbiPrism 7000 (Applied Biosystem) et le Takyon™ ROx SYBR MasterMix (Eurogenetec). Les données ont été analysées avec le programme 700 System SDS (Applied Biosystem) pour déterminer les valeurs de cycle seuil (Ct). L'expression de l'ARN messager (ARNm) a été calculée avec la méthode ΔCt et rapportée à l'expression de mHPRT.

### Western Blot

Les extractions de protéines et les expériences d'immunoblotting ont été réalisées comme précédemment décrit (Deschemin et al., 2015). Les anticorps utilisés sont les suivants: TLR4 anti-souris (sc-30002, Santa Cruz); anti-aquaporin4 (sc-20812, Santa cruz) et anti-β-actine (Sigma AC-74, product number A5316). Les anticorps secondaires utilisés étaient des anticorps anti-chèvre (Calbiochem) ou anti-lapin (Jackson ImmunoResearch Laboratories).

### Cytométrie de flux

Après avoir été saturées par des anticorps anti-CD32/CD16, les cellules ont été incubées avec des anticorps monoclonaux (mAb) dirigés contre mPDCA1 (JF05-1C2.4.1, conjugués à FITC), MHCII (IA/IE, 2G9 ou M5/114.15.2, FITC ou PE), CD103 (M290 ou 145-2D11, PE), CD86 (GL1, FITC) CD11b (M1/70, PerCP Cy5.5) ou CD11c (HL3, Biotin ou BV786). Pour le marquage des cellules T, les cellules ont été incubées avec des anticorps dirigés contre CD4 (L3T4 ou RM4-5, FITC ou PECy7), CD45.2 (104, A780), CD3 (145-2C11, PerCP Cy5.5) et CD8 (Ly2, 53-6.8, Biotine ou APC). Tous les mAb ont été obtenus de BD Biosciences sauf pour mPDCA1 (Miltenyi Biotec). La streptavidine conjuguée àl'APC- (BD biosciences) a été utilisée pour marquer l'anticorps-biotine. On a acquis au moins 2.10⁶ évènements avec un FACS Accuri ou un Fortessa (BD biosciences) qui ont ensuite été analysés avec le programme FlowJo Sofware v7.5 (Tree Star Inc).

### Dosage ELISA des cytokines et des Ig

Les cytokines IL-5, IL-10, IL-12p70, IL-17a and IFNγ (Mabtech) TSLP (Ready-SET-Go, eBiosciences) ont été quantifiées par ELISA en suivant les instructions du fabricant. Les IgE et les IgG1 ont été mesurées dans sérum des différentes souris (Ready-SET-Go, eBiosciences) en suivant les instructions du fabricant.

### Analyse statistique

Le test non paramétrique de Mann-Whitney (comparaison de 2 groupes, n ≥4), ou la méthode de Tukey pour les comparaisons multiples (> 2 groupes) ont été utilisés pour comparer les valeurs non appariées (GraphPadPrism software). La significativité est représentée: * p<0.05; ** p<0.01; *** p<0.001; and **** p<0.0001.

### • Effet sur l'évolution de l'asthme chez des souris inoculées avec la souche bactérienne CNCM 14969 inactivée par la chaleur

Les cultures de CNCM69 ont été obtenues comme décrit précédemment (108 cfu / mL) puis incubées ou non pendant 10 min à 100°C dans un bain marie. Après ce traitement, la culture a été étalée sur un milieu yhBHI Agar pendant 24h à 37°C pour vérifier l'efficacité du traitement thermique. Aucune bactérie n'a poussé après le traitement thermique, alors que les cultures ont poussé (après 24h à 37°C) pour les tubes n'ayant pas eu le choc thermique.

Le protocole d'administration de la souche et de déclenchement de l'asthme en présence d'HDM est celui décrit dans la figure 2A de la présente demande. La même procédure d'inoculation aux animaux comme celle décrite plus haut a été utilisée mais la souche CNCM69 inoculée a été inactivée ou non par un traitement à la chaleur.

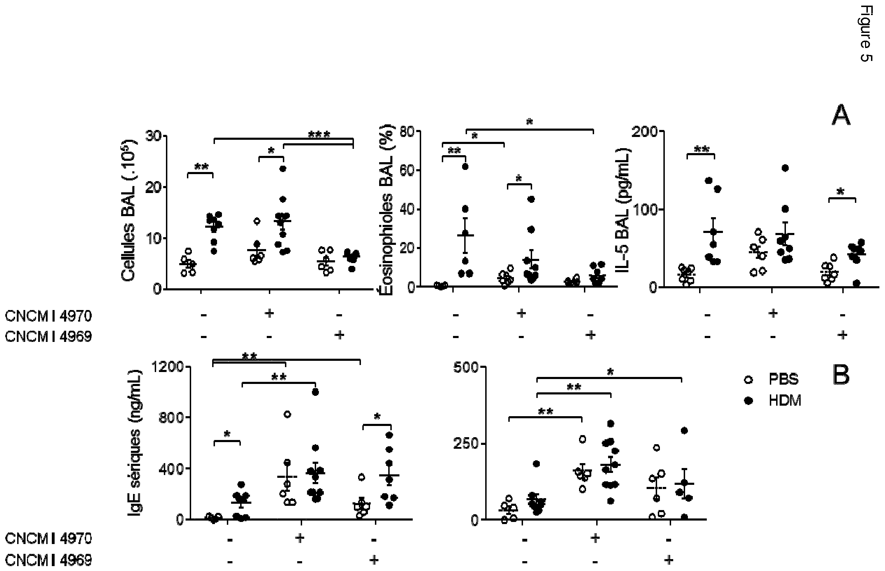

En outre, le taux de IgE sérique a été mesuré par dosage ELISA dans deux groupes de souris HDM inoculées avec la souche CNCM69 inactivée ou non par un traitement thermique. Le protocole de dosage est le même que celui décrit précédemment dans la légende de la figure 5B.

### 2. Résultats

Afin d'isoler des bactéries résidentes du poumon capables de moduler la susceptibilité de l'hôte face à des pathologies respiratoires, un modèle d'asthme induit chez des souriceaux a été utilisé. Il a d'abord été montré que les poumons des bactéries GF (germ free) sont fonctionnels malgré une homéostasie différente (Figure 1). Quand l'asthme allergique est induit, il n'est pas exacerbé dans des souris GF par rapport au contrôle (Figure 2).

Après l'induction de l'asthme allergique chez le souriceau, le microbiote du poumon est modifié. En effet, le nombre de bactéries cultivées par gramme de poumon augmente significativement lors du début de l'inflammation pulmonaire (Figure 3A). Certaines bactéries en particulier participent à cette augmentation, alors que d'autres bactéries ne varient pas significativement entre les animaux traités et contrôles (Figure 3B). Ce résultat prouve qu'une pathologie respiratoire comme l'asthme est associée à un changement des populations bactériennes résidentes du poumon.

Des bactéries primo-colonisatrices du poumon de souriceaux ont été isolées très tôt après la naissance (3 jours) et jusqu'au sevrage (3 semaines). Ces bactéries sont vivantes, et cultivables en absence ou en présence d'oxygène.

Une souche a été déposée à la CNCM sous le numéro CNCM I-4969. Cette bactérie possède des propriétés immuno-modulatrices. Lorsque ces bactéries primo-colonisatrices sont co-cultivées sur des coupes de poumons, elles induisent une réponse du tissu, forte et spécifique, puisque les réponses immunitaires mesurées par ELISA sont différentes en fonction de la bactérie utilisée (Figure 3C). Par exemple, la sécrétion de cytokines Th1 (IFNγ, IL-12p70), Th2 (TSLP) et anti-inflammatoire (IL-10) a été mesurée en présence de différentes bactéries, incluant CNCM I-4969 et d'autres bactéries respiratoires isolées au laboratoire (une souche de *E.coli* et un *Proteus mirabilis*).

Nos résultats démontrent que la bactérie CNCM 64969, qui est une bactérie primo-colonisatrice du poumon, possède des propriétés immuno-modulatrices (tableau ci-dessous).

| Souches | | Screening fonctionnel *ex vivo* (PCLS, figure 3) | Evolution *in vivo* pendant l'asthme (Figure 1) |
|---|---|---|---|
| A | N/A | Th1 | Pas de variation |
| B | N/A | Th1 | Pas de variation |
| CNCM I 4969 | Streptocoque | Th1 | Pas de variation |
| CNCM I 4970 | N/A | Th2 | augmente |

Cet aspect a été étudié plus en détail en testant la souche I-4969 *in vivo* chez la souris comme décrit ci-dessous.

Nos résultats montrent que les souriceaux recevant la bactérie CNCM 4969 sont protégés contre le retard de croissance dû à l'asthme (Figure 4).

Le tableau ci-dessous ainsi que la figure 8 montrent que cette protection contre le retard de croissance des souriceaux est aussi maintenue après l'inoculation de la souche CNCM 4969 désactivée par la chaleur.

| Méthode de Tukey pour comparaison multiple | Différence moyenne | q | Significatif ? P<0.05? | Résumé | 95% CI de la diff. |
|---|---|---|---|---|---|
| PBS vs HDM | 0.1584 | 8.100 | Yes | *** | 0.07633 to 0.2406 |
| PBS vs CNCM I 4969 + PBS | 0.01299 | 0.6639 | No | ns | -0.06912 to 0.09510 |
| PBS vs CNCM I 4969 + HDM | 0.07679 | 3.925 | No | ns | -0.005323 to 0.1589 |
| PBS vs CNCM I 4969 inactivée par la chaleur + PBS | 0.02078 | 1.062 | No | ns | -0.06133 to 0.1029 |
| PBS vs CNCM I 4969 inactivée par la chaleur + HDM | -0.01388 | 0.8631 | No | ns | -0.09899 to 0.06523 |
| HDM vs CNCM I 4969 + PBS | -0.1455 | 7.436 | Yes | *** | -0.2276 to -0.06335 |
| HDM vs CNCM I 4969 + HDM | -0.08166 | 4.174 | No | ns | -0.1638 to 0.0004534 |
| HDM vs CNCM I 4969 inactivée par la chaleur + PBS | -0.1377 | 7.037 | Yes | *** | -0.2198 to -0.05555 |
| HDM vs CNCM I 4969 inactivée par la chaleur + HDM | -0.1753 | 8.963 | Yes | *** | -0.2574 to -0.09322 |
| CNCM I 4969 + PBS vs CNCM I 4969 + HDM | 0.0638 | 3.261 | No | ns | -0.01831 to 0.1459 |
| CNCM I 4969 + PBS vs CNCM I 4969 inactivée par la chaleur + PBS | 0.007792 | 0.3984 | No | ns | -0.07432 to 0.08990 |
| CNCM I 4969 + PBS vs CNCM I 4969 inactivée par la chaleur + HDM | -0.02987 | 1.527 | No | ns | -0.1120 to 0.05224 |
| CNCM I 4969 + HDM vs CNCM I 4969 inactivée par la chaleur + PBS | -0.05601 | 2.863 | No | ns | -0.1382 to 0.02610 |
| CNCM I 4969 + HDM vs CNCM I 4969 inactivée par la chaleur + HDM | -0.09367 | 4.788 | Yes | * | -0.1758 to -0.01156 |
| CNCM I 4969 inactivée par la chaleur + PBS vs CNCM I 4969 inactivée par la chaleur + HDM | -0.03766 | 1.925 | No | ns | -0.1198 to 0.04445 |

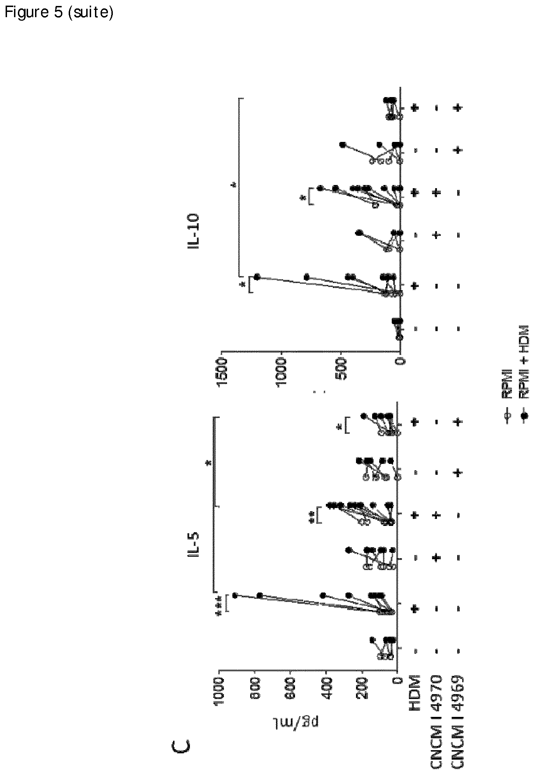

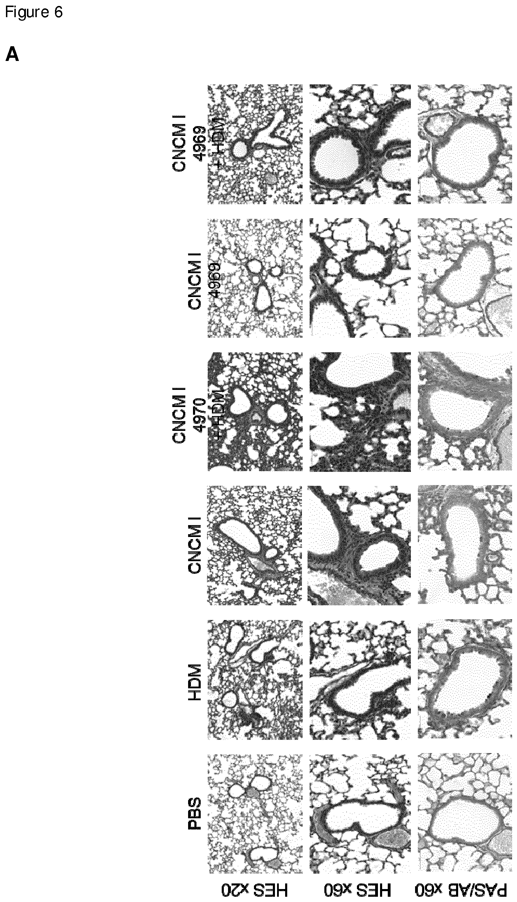

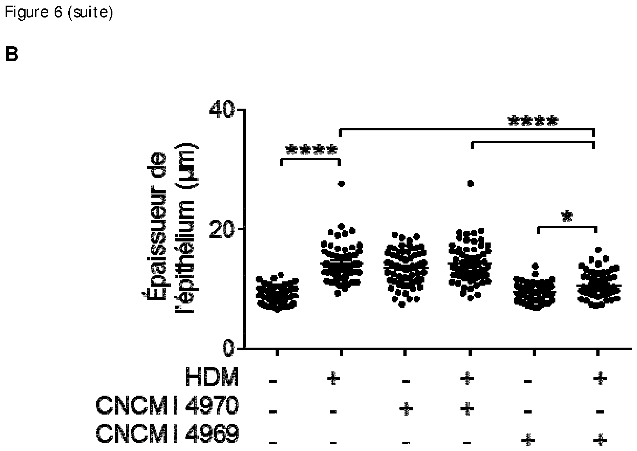

Nous avons ensuite analysé plusieurs paramètres immunitaires et inflammatoires. Nous avons observé que la bactérie CNCM 4970 augmente la quantité d'IgE dans le sang (ce type d'immunoglobuline est augmenté lors de réactions allergiques) (Figure 5B). En restimulant *in vitro* les cellules des ganglions respiratoires avec du milieu seul ou du milieu contenant de l'HDM, nous avons constaté que les cytokines Th2 sont significativement moins augmentées pour ceux recevant la CNCM 4969 (Figure 5C). D'autre part, l'inflammation du poumon est atténuée en présence de la CNCM I-4969, comme le montrent divers paramètres physiologiques (Figure 6). En revanche, la souche CNCM I-4970 exacerbe cette inflammation. Nos conclusions sont que la bactérie CNCM 4969 diminue la signature Th2, et protège contre le retard de croissance, ce qui lui confère des propriétés protectrices contre l'asthme.

En outre, les résultats obtenus après le dosage du taux d'IgE sanguin chez des souris inoculées avec la souche CNCM 4969 inactivée par la chaleur montrent que ce taux ne diminue pas, suggérant que la signature Th2 n'est pas augmentée (Figure 9).

En ce qui concerne les bactéries CNCM 4969 inactivées par la chaleur, nous pouvons conclure également que celles-ci permettent de conférer des propriétés protectrices contre l'asthme.

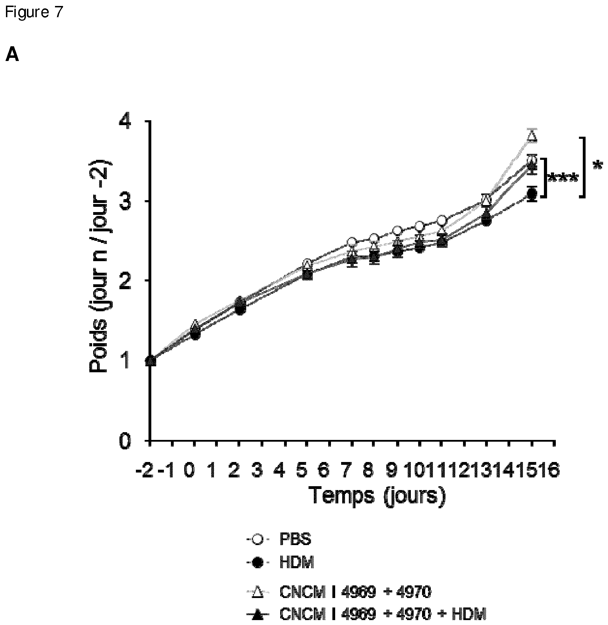

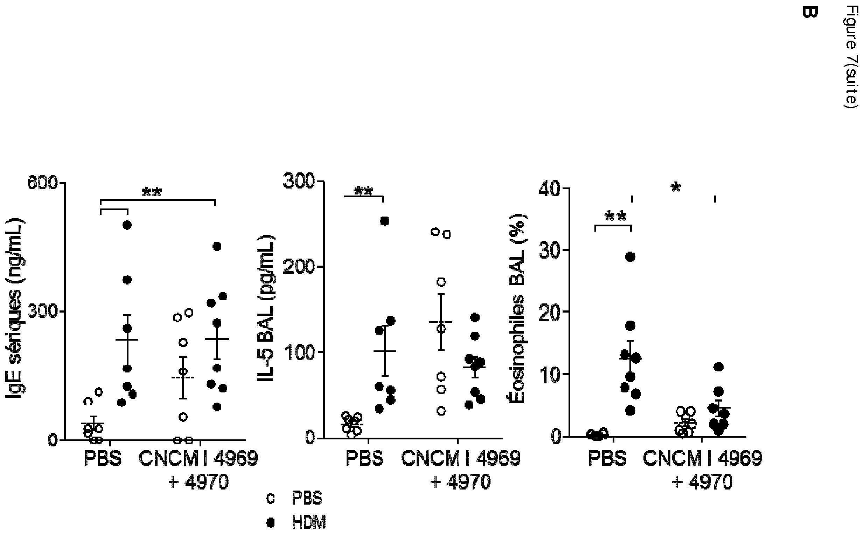

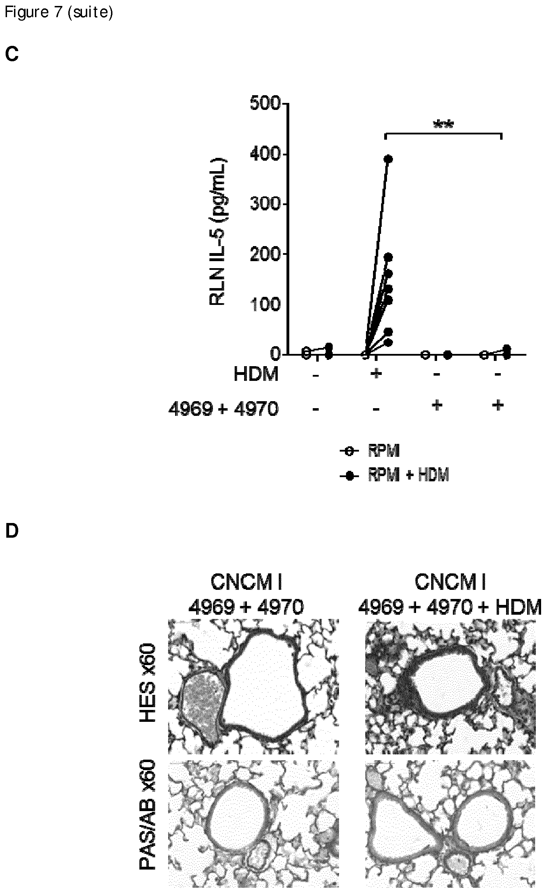

La seconde expérience que nous avons réalisée confirme les courbes de poids de la première. Pour cette 2^{nd} expérience, nous avons eu plus de souriceaux et avons pu ajouter des groupes : les deux bactéries ensemble (Figure 7) ; les bactéries inactivées à la chaleur. Nos résultats basés sur les courbes de poids et les dosages des IgE dans le sang montrent que les deux bactéries administrées ensemble induisent un profil intermédiaire par rapport aux résultats observés avec CNCM 4969 ou 70 seules.

### REFERENCES BIBLIOGRAPHIQUES

1. Sibley CD, Grinwis ME, Field TR, Eshaghurshan CS, Faria MM, et al. (2011) Culture Enriched Molecular Profiling of the Cystic Fibrosis Airway Microbiome. PLoSONE 6: e22702.
2. Gollwitzer E, Saglani S, Trompette A, Yadava K, Sherburn R, et al. (2014) Lung microbiota promotes tolerance to allergens in neonates via PD-L1. Nature medicine 20: 642-647.
3. Erb-Downward JR, Thompson DL, Han MK, Freeman CM, McCloskey L, et al. (2011) Analysis of the Lung Microbiome in the "Healthy" Smoker and in COPD. PloSONE 6.
4. Erb-Downward JR, Huffnagle GB, and Martinez FJ (2012) The microbiota in Respiratory Disease. American Journal of Respiratory and Critical Care Medicine 185: 1037-1038.
5. Yun Y, Srinivas G, Kuenzel S, Linnenbrink M, Alnahas S, et al. (2014) Environmentally determined differences in the murine lung microbiota and their relation to alveolar architecture. PLoS One 9: e1134.
6. Jang S, Kim H, Kim Y, Kang M, Kwon J, et al. (2012) Asthma Prévention by Lactobacillus Rhamnosus in a Mouse Model is Associated With CD4(+)CD25(+)Foxp3(+) T Cells. Allergy, asthma & immunol research 4: 150-156.
7. Kim H, Kim Y, Lee S, Kang M, Yu H, et al. (2013) Effects of Lactobacillus rhamnosus on asthma with an adoptive transfer of dendritic cells in mice. Journal of applied microbiology 115: 872-879.

## Revendications

1. Souche *d'Enterococcus* sp. déposée à la Collection nationale des cultures de microorganismes (CNCM, 25 rue du Docteur Roux, 75724 Paris Cedex 15, France) sous le numéro I-4969.

2. Composition pharmaceutique comprenant la souche de la revendication 1 et au moins un excipient pharmaceutiquement acceptable.

3. La composition de la revendication 2 **caractérisée en ce que** la souche est inactivée.

4. La composition de la revendication 3 **caractérisée en ce que** la souche est inactivée par la chaleur.

5. Composition pharmaceutique **caractérisée en ce qu'**elle comprend un extrait consistant en un lysat de la souche de la revendication 1.

6. La composition de l'une quelconque des revendications 2 à 5 pour utilisation dans le traitement et/ou la prévention des maladies respiratoires.

7. La composition pour une utilisation selon la revendication 6 **caractérisée en ce que** l'expression des cytokines Th2 n'est pas augmentée ou est diminuée chez un patient traité par rapport à un patient non traitée par ladite souche.

8. La composition pour une utilisation selon l'une quelconque des revendications 6 ou 7, **caractérisée en ce que** le patient traité est un enfant.

## Patentansprüche

1. *Enterococcus sp.* Stamm, der bei der Collection Nationale des Cultures de Microorganismes (CNCM, 25 Rue du Docteur Roux, 75724 Paris Cedex 15, Frankreich) unter der Nummer I-4969 hinterlegt wurde.

2. Pharmazeutische Zusammensetzung, die den Stamm nach Anspruch 1 und mindestens einen pharmazeutisch annehmbaren Träger umfasst.

3. Zusammensetzung nach Anspruch 2, **dadurch gekennzeichnet, dass** der Stamm inaktiviert ist.

4. Zusammensetzung nach Anspruch 3, **dadurch gekennzeichnet, dass** der Stamm hitzeinaktiviert ist.

5. Pharmazeutische Zusammensetzung, **dadurch gekennzeichnet, dass** sie einen Extrakt umfasst, der aus einem Lysat des Stamms nach Anspruch 1 besteht.

6. Zusammensetzung nach einem der Ansprüche 2 bis 5 zur Verwendung bei der Behandlung und/oder der Vorbeugung von Atemwegserkrankungen.

7. Zusammensetzung für eine Verwendung nach Anspruch 6, **dadurch gekennzeichnet, dass** die Th2-Zytokinexpression bei einem behandelten Patienten im Vergleich zu einem nicht mit dem Stamm behandelten nicht erhöht ist oder verringert ist.

8. Zusammensetzung für eine Verwendung nach einem der Ansprüche 6 oder 7, **dadurch gekennzeichnet, dass** der behandelte Patient ein Kind ist.

## Claims

1. *Enterococcus* sp. strain deposited with the *Collection nationale des cultures de microorganismes* (CNCM, 25 rue du Docteur Roux, 75724 Paris Cedex 15, France) under number 1-4969.

2. Pharmaceutical composition comprising the strain of claim 1 and at least one pharmaceutically acceptable excipient.

3. The composition of claim 2 **characterised in that** the strain is inactivated.

4. The composition of claim 3 **characterised in that** the strain is heat-inactivated.

5. Pharmaceutical composition **characterised in that** it comprises an extract consisting of a lysate of the strain of claim 1.

6. The composition of any one of claims 2 to 5 for use in the treatment and/or prevention of respiratory diseases.

7. The composition for use according to claim 6 **characterised in that** the expression of Th2 cytokines is not increased or is decreased in a patient treated with said strain relative to a patient not treated with said strain.

8. The composition for use according to any one of claims 6 or 7, **characterised in that** the treated patient is a child.
